# EUROPEAN PATENT SPECIFICATION

(11) **EP 1 332 757 B1**
(45) Date of publication and mention of the grant of the patent: **13.06.2012**
(21) Application number: 03076054.0
(22) Date of filing: 24.05.1999
(51) Int. Cl.: A61K 9/28, A61K 9/20, A61K 31/535

(54) **Efavirenz compressed tablet formulation**
Efavirenz enthaltende gepresste Tablettenformulierung
Formulation de comprimé d' efavirenz

(30) Priority: 27.05.1998 US 86921 P; 21.07.1998 GB 9815800
(43) Date of publication of application: 06.08.2003
(62) Divisional of application: 99925793.4
(73) Proprietor: Merck Sharp & Dohme Corp., Rahway, NJ 07065 (US)
(72) Inventor: Batra, Udit, Rahway, New Jersey 07065 (US); Higgins, Raymond J., Rahway, New Jersey 07065 (US); Thompson, Karen C., Rahway, New Jersey 07065 (US); Katdare, Ashok V., Rahway, New Jersey 07065 (US)
(74) Representative: Horgan, James Michael Frederic

(56) References cited:
- WO-A-95/20389
- WO-A-96/37457
- WO-A-98/52570
- WO-A1-99/51239

## Description

### BACKGROUND OF THE INVENTION

This invention relates to a compressed tablet formulation for efavirenz, which is 50 percent by weight drug loaded and can optionally be film coated. Efavirenz is a non-nucleoside reverse trancriptase inhibitor being studied clinically for use in the treatment of HIV infections and AIDS. A process for the manufacture of the compressed tablet is also disclosed.

The synthesis of efavirenz and structurally similar reverse transcriptase inhibitors are disclosed in US Patents 5,519,021, 5,663,169, 5,665,720 and the corresponding PCT International Patent Application WO 95/20389, which published on August 3, 1995. Additionally, the asymmetric synthesis of an enantiomeric benzoxazinone by a highly enantioselective acetylide addition and cyclization sequence has been described by Thompson, et al., Tetrahedron Letters 1995, 36, 8937-8940, as well as the PCT publication, WO 96/37457, which published on November 28, 1996.

Additionally, several applications have been filed which disclose various aspects of the synthesis of(-)-6-chloro-4-cyclopropyl-ethynyl-4-trifluoromethyl-1,4-dihydro-2H-3,1-benzoxazin-2-one including: 1) a process for making the chiral alcohol, U.S.S.N. 60/035,462, filed 14 January 1997; 2) the chiral additive, U.S.S.N. 60/034,926, filed 10 January 1997; 3) the cyclization reaction, U.S.S.N. 60/037,059, filed 12 February 1997; and the anti-solvent crystallization procedure, U.S.S.N. 60/037,385 filed 5 February 1997 and U.S.S.N. 60/042,807 filed 8 April 1997.

WO-A-9951239 (Du Pont Pharmaceuticals Company) designates AT, BE, CH, DE, DK, ES, FR, GB, GR, IE, IT, LI, LU, NL, PT, SE and FI and is citeable under Art. 54(3) EPC for these states only. It discloses compressed tablets comprising efavirenz and greater than 10% by weight of super disintegrant. The efavirenz used in the tablets is made by the process of US-A-5519021 which produces crystalline efavirenz.

The compressed tablet is an improved formulation which allows one to utilize a tablet over a capsule. The compressed tablet has been demonstrated to have comparable bioavailability data to that seen with the capsule. The key feature of the formulation is the use of a superdisintegrant and disintegrant intragranularly to achieved a bioequivalent formulation. The compressed tablet form was difficult to manage as efavirenz is fragile and the drug loses crystallinity upon compression. This was overcome by adding lactose extragranularly.

The present invention provides a compressed tablet comprising:
efavirenz, filler/disintegrant, superdisintegrant, binder, surfactant, a diluent/compression aid which comprises lactose, lubricant, and solvent, wherein the efavirenz is crystalline, which tablet is obtainable by a granulation process in which the superdisintegrant and disintegrant are added intragranularly and the lactose is added extragranularly and which tablet:
   (i) comprises from 1 to 75% of efavirenz by weight of the total composition of the compressed tablet;
      (a) providing the tablet comprises no more than 10% by weight of the total weight of superdisintegrant; or
      (b) wherein the binder is a hydroxypropylcellulose; or
   (ii) contains 600 mg of efavirenz in the total composition of the compressed tablet.

The efivarenz concentration can be varied from about 1 to about 75% by changing the concentration of remaining excipients. Efavirenz may be about 50% by weight of the total composition of the compressed tablet. Furthermore, changing the tooling can give a wide range of doses, e.g. a 20 mg dose in a 40 mg tablet, a 300 mg dose in a 600mg tablet, or a 600 mg dose in a 1200 mg compressed tablet, with the same composition. Removing the lactose from the formulation gives about 70% drug in the formulation giving a 600 mg dose in a 860 mg compressed tablet. These variations are very straightforward to effect. This formulation will allow one to formulate efavirenz as a single 600 mg dose as an 860 mg compressed tablet, where as a capsule formulation requires the administration of at least two capsules to dose with 600 mg of efavirenz.

The invention contemplates the use of any pharmaceutically acceptable fillers/compression aids, disintegrants, super-disintegrants, lubricants, binders, surfactants, film coatings, and solvents. Examples of these components are set forth below and are described in more detail in the Handbook of Pharmaceutical Excipients, Second Edition, Ed. A. Wade and P.J. Weller, 1994, The Pharmaceutical Press, London, England.

Fillers and compression aid concentrations can be varied between about 5% to about 80% to complement the drug amount. Examples of fillers/compression aids include: lactose, calcium carbonate, calcium sulfate, compressible sugars, dextrates, dextrin, dextrose, calcium phosphate, kaolin, magnesium carbonate, magnesium oxide, maltodextrin mannitol, powdered cellulose, pregelatinized starch, and sucrose.

Examples of disintegrants include: alginic acid, carboxymethylcellulose calcium, carboxymethylcellulose sodium, colloidal silicon dioxide, croscarmellose sodium, crospovidone, guar gum, magnesium aluminum silicate, methylcellulose, microcrystalline cellulose, polyacrilin potassium, powdered cellulose, pregelatinized starch, sodium alginate and starch.

Examples of fillers (also referred to as a diluent) include: calcium carbonate, calcium sulfate, compressible sugars, confectioner's sugar, dextrates, dextrin, dextrose, dibasic calcium phosphate dihydrate, glyceryl palmitostearate, hydrogenated vegetable oil (type I), kaolin, lactose, magnesium carbonate, magnesium oxide, maltodextrin, mannitol, polymethacrylates, potassium chloride, powdered cellulose, pregelatinized starch, sodium chloride, sorbitol, starch, sucrose, sugar spheres, talc and tribasic calcium phosphate.

Superdisintegrant concentration can be varied between about 1% to about 20% to complement the drug amount and obtain reasonable dissolution. Examples of super-disintegrants include the disintegrants listed above, carboxymethylcellulose sodium, croscarmellose sodium, povidone, guar gum, polacrilin potassium, and pregelatinized starch.

Binder concentration can be varied between 1 and 10 % to complement the drug amount. Examples of binders include: acacia, alginic acid, carbomer, carboxymethylcellulose sodium, dextrin, ethylcellulose, gelatin, guar gum, hydrogenated vegetable oil (type I), hydroxyethyl cellulose, hydroxypropyl cellulose, hydroxypropyl methylcellulose, liquid glucose, magnesium aluminaum silicate, maltodextrin, methylcellulose, polymethacrylates, povidone, pregelatinized starch, sodium alginate, starch, and zein.

Examples of surfactants comprises anionic and cationic surfactants, such as sodium lauryl sulfate, docusate sodium (dioctyl sulfosuccinate sodium salt), benzalkonium chloride, benzethonium chloride, and cetrimide (alkyltrimethylammonium bromide, predominantly C₁₄ alkyl).

Examples of lubricants include: calcium stearate, glyceryl monostearate, glyceryl palmitostearate, hydrogenated castor oil, hydrogenated vegetable oil, light mineral oil, magnesium stearate, mineral oil, polyethylene glycol, sodium benzoate, sodium lauryl sulfate, sodium stearyl fumarate, stearic acid, talc and zinc stearate.

Examples of solvent comprises: water, ethanol or mixtures thereof.

The compressed tablet can also be film coated. Film coat concentration can be varied up to about 10 % to complement the drug amount, and preferably about 3.1% to about 3.3%. Film coating suspensions include combinations of one, two or three of the following components: carboxymethylcellulose sodium, carnauba wax, cellulose acetate phthalate, cetyl alcohol, confectioner's sugar, ethyl cellulose, gelatin, hydroxyethyl cellulose, hydroxypropyl cellulose, hydroxypropyl methylcellulose, liquid glucose, maltodextrin, methyl cellulose, microcrystalline wax, Opadry and Opadry II, polymethacrylates, polyvinyl alcohol, shellac, sucrose, talc, titanium dioxide, and zein.

The preferred filler/disintegrant is microcrystalline cellulose. The preferred superdisintegrant is croscarmellose sodium. The preferred binder is hydroxypropyl cellulose. A preferred surfactant is sodium lauryl sulfate. The preferred diluent/compression aid is lactose hydrous spray dried. The preferred lubricant is magnesium stearate. The preferred solvent for formulating this compressed tablet is water. The preferred film coating comprises: hydroxypropylcellulose, hydroxypropyl methylcellulose, and titanium dioxide.

The 300 mg film coated efavirenz tablet contains:

| **Ingredient** | **Amt per tablet** | **Percent w/w** |
|---|---|---|
| **Core Tablet:** | | |
| efavirenz | 300 mg | 50 |
| microcrystalline cellulose NF | 120 mg | 20 |
| hydroxypropyl cellulose LF NF | 19.2 mg | 3.2 |
| croscarmellose sodium | 30 mg | 5 |
| sodium lauryl sulfate | 6 mg | 1 |
| lactose hydrous spray dried (EG) | 118.8 mg | 19.8 |
| magnesium stearate (EG) | 6 mg | 1 |
| **Film Coating Material per Tablet:** | 3.1% by wt | |
| hydroxypropyl cellulose LF NF | 8.05 mg | 1.4 |
| hydroxypropyl methylellulose USP 6CPS | 8.05 mg | 1.4 |
| titanium dioxide USP | 3.1 mg | 0.3 |
| **Tablet Weight:** | 619.2mg | |

A process for the preparation of a 50 % drug loaded compressed tablet comprising the following steps:
(a) blending cristalline efavirenz with a filler/disintegrant, super-disintegrant, binder and surfactant;
(b) adding at least 1.1% by weight of water per weight of efavirenz to wet granulate the blended mixture to agglomerate the mixture;
(c) drying the granulated mixture to a moisture content of about 0% to about 10%;
(d) milling the dried mixture to granulate to a uniform size;
(e) blending the milled mixture with a filler/compression aid which comprises lactose;
(f) lubricating the blended mixture with a lubricant; and
(g) compressing the lubricated mixture to a compressed tablet of the desired shape.

The process as recited above which comprises the additional step of film coating the compressed tablet with a film coating suspension to produce the desired film coated compressed tablet.

The process as recited above wherein the granulated mixture is dried to a moisture content of about 2% to about 5%.

A process for the preparation of a 50 % drug loaded compressed tablet comprising the following steps:
(a) blending crystaline efavirenz with microcrystalline cellulose, sodium lauryl sulfate, hydroxypropyl cellulose and croscarmellose sodium;
(b) adding at least 1.1 weight % water per weight of efavirenz to wet granulate the blended mixture for about 3 minutes to about 8 minutes to agglomerate the mixture;
(c) drying the granulated mixture to a moisture content of about 2% to about 5%;
(d) milling the dried mixture to a granulate of about 250µm to about 75µm;
(e) blending the milled mixture with lactose;
(f) lubricating the blended mixture with magnesium stearate;
(g) compressing the lubricated mixture to a compressed tablet of the desired shape; and
(h) film coating the compressed tablet with a film coating suspension to about 3.1% to about 8.8% of weight of compressed tablet.

The process as recited above wherein the blended mixture is wet granulated for about 6 minutes.

The process as recited above wherein the film coating suspension comprising hydroxypropylcellulose, hydroxypropyl methylcellulose, and titanium dioxide.

Wet granulation can be conducted using granulator mixers, such as a Fielder 10 L high shear granulator mixer, a drum or pan granulator, and a fluid bed granulator, Granulation can also be achieved by conducting dry granulation (without water) using a roller compaction process.

The drying step can be conducted using a Glatt WST-15 fluid bed drier or a tray drier.

The milling step can be conducted using mills such as a Cornil or a Fitz mill.

The lubricating and blending steps can be conducted in a V-blender or a ribbon blender.

The compression step to form the tablet can be done a variety of presses including a beta press, single station F-press, the 6-station Korsh, etc.

Film coating can be performed in a Glatt Column coater, a smaller Hi-coater (9"- 12 " pan), etc.

The formulation also is bioequivalent to a capsule with a smaller dose (200 mg), and more bioavailable than other tablet compositions. The advantages over the capsule include robust processing and sorting steps, smaller size with a larger dose, and market preference. The tablet composition also overcomes the expect loss of crystallinity of efavirenz by adding the lactose extra-granularly while maintaining the dissolution profile.

The increased drug loading often compromises the dissolution profile of the drug. This hurdle was overcome by including the super-disintegrant intragranularly, as well as the disintegrant intragranularly. The lactose was added extra-ganularly to maintain the crystallinity of efavirenz.

This formulation was determined to be bioequivalent to the capsule formulation being used in clinical trials. The wet granulation process has been used to optimize the formulation such that about 80% dissolution of the drug occurs within 10 minutes in a 1% Sodium Dodecyl sulfate (SDS) solution, while stirring at a 50 rpm paddle speed.

Preparation of (-)-6-chloro-4-cyclopropylethynyl-4-trifluoromethyl-1,4-dihydro-2H-3,1-benzoxazin-2-one (currently referred to by its generic name efavirenz or code name DMP-266).

Scheme 1 outlines the key steps in the synthesis of (-)-6-chloro-4-cyclopropylethynyl-4-trifluoromethyl-1,4-dihydro-2H-3,1-benzoxazin-2-one (efavirenz). The chiral addition step allows for the enantioselective addition of the cyclopropylacetylide across the trifluoromethylketone of 1. The p-methoxybenzyl (PMB)-protected amino alcohol, 2, produced is then deprotected to give the amino alcohol, 3. The amino alcohol is then cyclized using a chloroformate and base to give efavirenz.

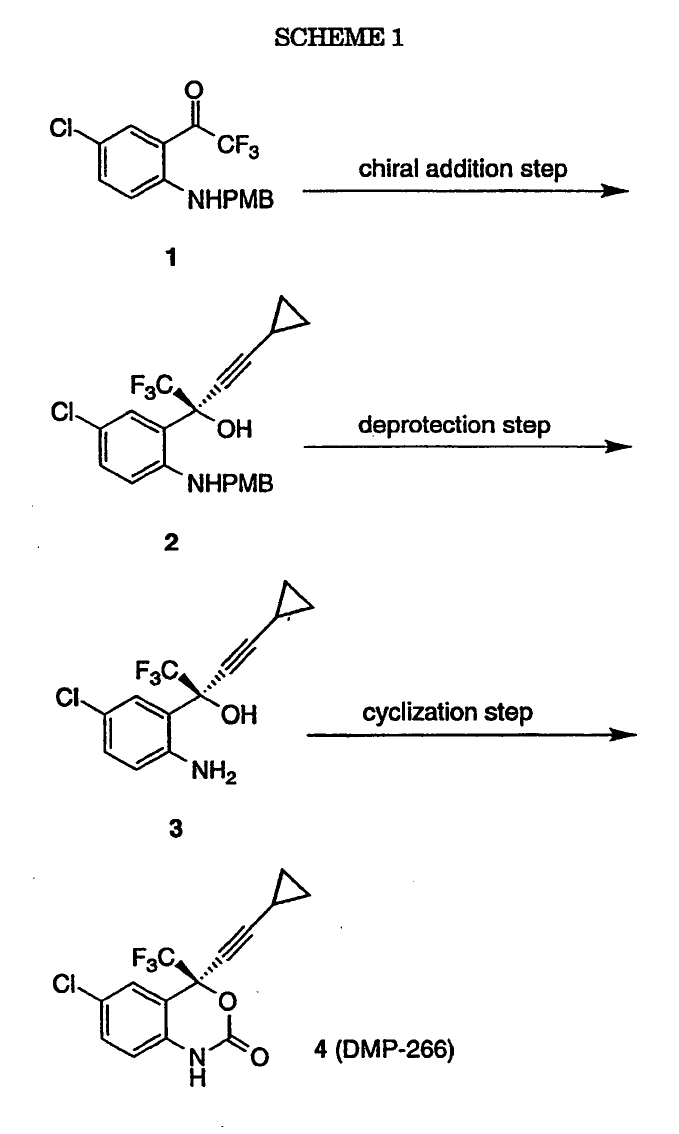

Scheme 2 outlines the preparation of efavirenz using an alternative process which is a chiral addition reaction. The new chiral addition reaction allows for the elimination of the protection-deprotection sequence as outlined in Scheme 1.

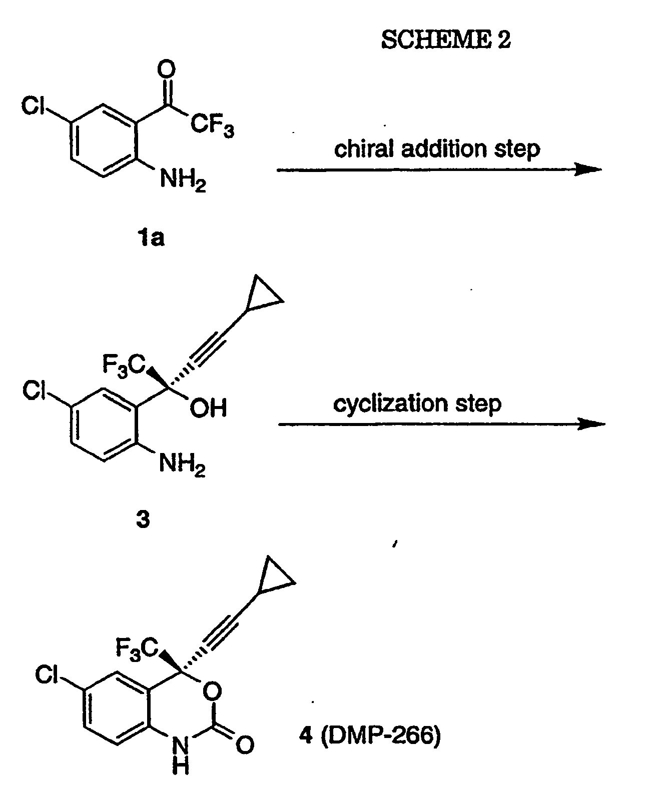

Scheme 3 describes the process for the synthesis of the chiral intermediate used in the preparation of efavirenz. This reaction has been demonstrated to work using about 1.2 equivalents of cyclopropylacetylene and chiral additive, much lesss than the prior methods. The numerous chiral additives have been run and give high yields with a commerically available chiral ligand, such as N-methyl ephedrine and N-pyrrolidinyl norephedrine.

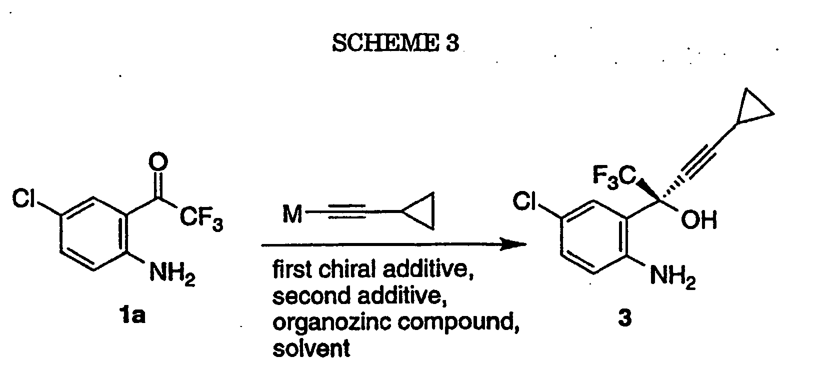

The cyclization of the amino alcohol, **3** to produce the 1,4-dihydro-2H-3,1-benzoxazin-2-one, **4** is outlined in Scheme 4 below. The reaction can be carried out as a one-step process, or alternatively a two step process with the potential isolation of the intermediate carbamate, **5** depending upon the chloroformate utilized. It has been demonstrated that the aryl chloroformates form less stable carbamates such that when they are treated with aqueous base they cyclize to the product, in a one-step process. The alkyl chloroformate, alternatively, provides an alkyl carbamate, a key intermediate capable of being isolated and purified prior to carrying out the cyclization step. Based upon the stability of the alkyl carbamates, a viable two step process for the preparation of efavirenz has been developed which comprises the formation of the alkyl carbamate intermediate, **5** followed by the cyclization of the carbamate to give the desired product, **4**. Additionally, it has been demonstrate that phosgene can also be used.

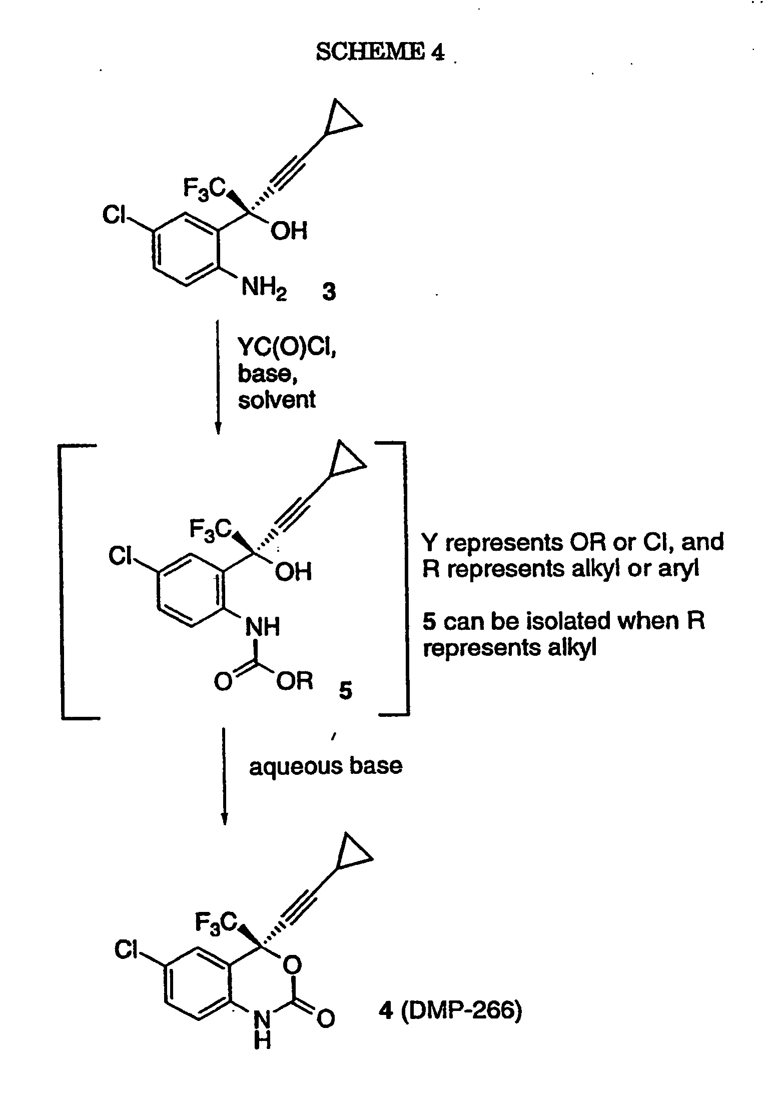

The compressed tablet is formulated following the sequence of steps outlined in Scheme 5.

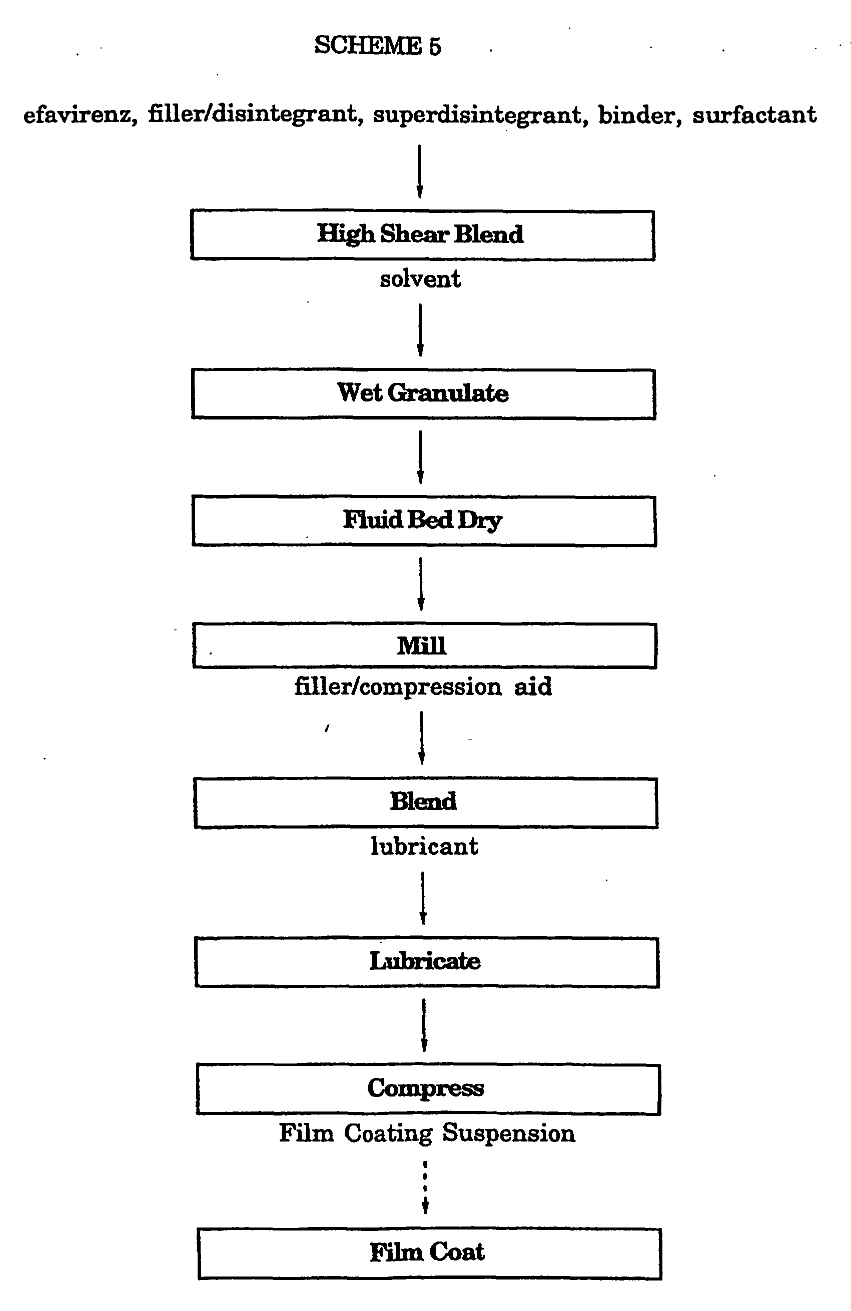

The following examples are meant to be illustrative of the present invention. These examples are presented to exemplify the invention and are not to be construed as limiting the scope of the invention.

### EXAMPLE 1

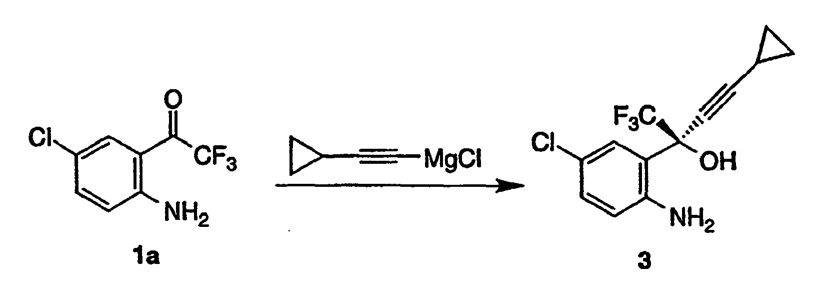

| **Materials** | Amount | Mol | MW |
|---|---|---|---|
| Ketone 1a | 1.00 kg g | 4.47 | 223.58 |
| (1R, 2S)-N-pyrrolidinyl norephedrine | 1.35 kg | 6.58 | 205.30 |
| cyclopropyl acetylene | 361.9 g | 5.47 | 66.10 |
| *n*-BuMgCl (2.0 M in THF) | 2.68 L | 5.37 | |
| 2,2,2-trifluoroethanol (99%) | 429.5 g | 4.29 | 100.04 |
| ZnEt₂ (0.892 M in hexane) | 6.02 L | 5.37 | |
| THF | 9.36 L | | |
| 30% K₂CO₃ | 550 mL | | |
| 30% citric acid | 2.0 L | | |
| Toluene (for crystallization, 2 mL/g of 4) | 2.6 L | | |
| Heptane (for crystallization, 4 mL/g of 4) | 5.2 L | | |

To a solution of trifluoroethanol and (1R, 2S)-N-pyrrolidinyl norephedrine in THF (9 L) under nitrogen is added a solution of diethylzinc in hexane at 0 °C slowly enough to keep the temperature below 30 °C. The mixture is stirred at room temperature for 0.5 - 1 h. In another dry flask a solution of chloromagnesium cyclopropyl acetylide is prepared as follows: To neat cyclopropyl acetylene at 0 °C is added a solution of *n*-butylmagnesium chloride slowly enough to keep the internal temperature ≤ 30 °C. The solution is stirred at 0 °C for - 40 min and transfered to the zinc reagent *via* cannula with 0.36 L of THF as a wash. The mixture is cooled to -10 °C and ketoaniline **1a** is added. The mixture is stirred at -2 to -8 °C for 35 h, warmed to room temperature, stirred for 3 h, and quenched with 30% potassium carbonate over 1.5 h. The mixture is stirred for 4 h and the solid is removed by filtration and washed with THF (2 cake volume). The wet solid still contains ∼18 wt% of pyrrolidinyl norephedrine and is saved for further study. The filtrate and wash are combined and treated with 30% citric acid. The two layers are separated. The organic layer is washed with water (1.5 L). The combined aqueous layers are extracted with 2.5 L of toluene and saved for norephedrine recovery. The toluene extract is combined with the organic solution and is concentrated to - 2.5 L. Toluene is continuously feeded and distilled till THF is not detectable by GC. The final volume is controlled at 3.9 L. Heptane (5.2 L) is added over 1 h. The slurry is cooled to 0 °C, aged for 1 h, and filtered. The solid is washed with heptane (2 cake volume) and dried to give 1.234 Kg (95.2% yield) of amino alcohol 3 as a white crystalline.' The material is 99.8 A% pure and 99.3% ee.

### EXAMPLE 2

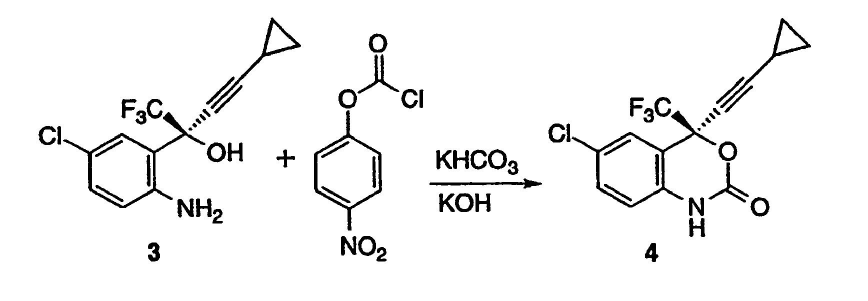

| | FW | g | mL | mmol | equiv |
|---|---|---|---|---|---|
| amino alcohol 8 | 289 | 100 | | 346 | 1 |
| 4-nitrophenylchloroformate | 201.6 | 73.2 | | 363 | 1.05 |
| KHCO₃ | 100 | 45 | | 450 | 1.3 |
| 2NKOH | 56 | | 346 | 692 | 2.0 |
| H₂O | | | 654 | | |
| MTBE | | | 500 | | |

To a three necked round bottom flask, equipped with a mechanical stirrer, nitrogen line, and thermocouple, was charged the solid amino alcohol **3,** MTBE (500 mL), and aqueous KHCO₃ (45 g in 654 mL H₂O). Solid 4-nitrophenyl chloroformate was added, in 4 batches, at 25°C. During the addition the solution pH was monitored. The pH was maintained between 8.5 and 4 during the reaction and ended up at 8.0. The mixture was stirred at 20-25°C for two hours. Aqueous KOH (2N) was added over 20 minutes, until the pH of the aqueous layer reached 11.0.

The layers were separated and 500 mL brine was added to the MTBE layer. 0.1 N Acetic acid was added until the pH was 6-7. The layers were separated and the organic phase was washed with brine (500 mL). At this point the mixture was solvent switched to EtOH/IPA and crystallized as recited in Examples 5 and 6.

### EXAMPLE3

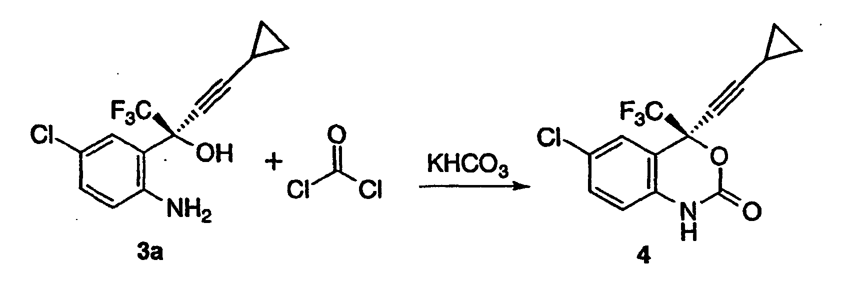

| | FW | g | mL | mmol | equiv |
|---|---|---|---|---|---|
| amino alcohol **3a** | 289 | 100 | | 346 | 1 |
| phosgene (20 wt% in toluene) | 99 | 41 | 216 | 415 | 1.2 |
| KHCO₃ | 100 | 86.5 | | 865 | 2.5 |
| H₂O | | | 500 | | |
| Toluene | | | 500 | | |

To a three necked round bottom flask, equipped with a mechanical stirrer, nitrogen line, and thermocouple, was charged the solid amino alcohol **3a,** toulene (500 mL), and aqueous KHCO₃ (86.5 g in 500 mL H₂O). Phosgene solution in toulene was added at 25°C, and the mixture was stirred at 20-25°C for two hours.

The layers were separated and the organic phase was washed with brine (500 mL). At this point the mixture was solvent switched to EtOH/IPA and crystallized as recited in Examples 5 and 6.

### EXAMPLE 4

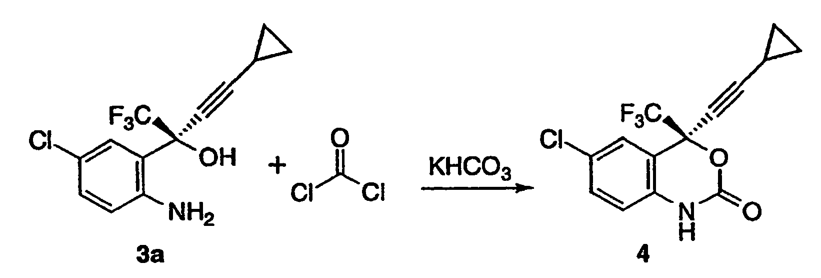

| | FW | g | mL | mmol | equiv |
|---|---|---|---|---|---|
| amino alcohol **3a** | 289 | 100 | | 346 | 1 |
| phosgene (gas) | 99 | | | | |
| KHCO₃ | 100 | 86.5 | | 865 | 2.5 |
| H₂O | | | 500 | | |
| MTBE | | | 500 | | |

To a three necked round bottom flask, equipped with a mechanical stirrer, nitrogen line, and thermocouple, was charged the solid amino alcohol **3a,** MTBE (500 mL), and aqueous KHCO₃ (86.5 g in 500 mL H₂O). Phosgene gas was slowly passed into the solution at 25°C, until the reaction was complete.

The layers were separated and the organic phase was washed with brine (500 mL). At this point the mixture was solvent switched to EtOH/IPA and crystallized as recited in Examples 5 and 6.

### EXAMPLE 5

Crystallization of efavirenz from 30% 2-Propanol in Water using a ratio of 15 ml solvent per gram efavirenz Using Controlled Anti-Solvent Addition on a 400 g Scale.

400 g. of efavirenz starting material is dissolved in 1.8 L of 2-propanol. The solution is filtered to remove extraneous matter. 1.95 L of deionized (DI) water is added to the solution over 30 to 60 minutes. 10 g. to 20 g. of efavirenz seed (Form II wetcake) is added to the solution. The seed bed is aged for 1 hour. The use of Intermig agitators is preferred to mix the slurry. If required (by the presence of extremely long crystals or a thick slurry), the slurry is wet-milled for 15 - 60 seconds. 2.25 L of DI water is added to the slurry over 4 to 6 hours. If required (by the presence of extremely long crystals or a thick slurry), the slurry is wet-milled for 15 - 60 seconds during the addition. The slurry is aged for 2 to 16 hours until the product concentration in the supernatant remains constant. The slurry is filtered to isolate a crystalline wet cake. The wet cake is washed with 1 to 2 bed volumes of 30 % 2-propanol in water and then twice with 1 bed volume of DI water each. The washed wet cake is dried under vacuum at 50°C.

### EXAMPLE 6

Crystallization of efavirenz from 30% 2-Propanol in Water using a ratio of 15 ml solvent per gram efavirenz Using a Semi-Continuous Process on a 400 g Scale.

400 g. of efavirenz starting material is dissolved in 1.8 L of 2-propanol. A heel slurry is produced by mixing 20 g. of Form II efavirenz in 0.3 L of 30 % (v/v) 2-propanol in water or retaining part of a slurry from a previous crystallization in the crystallizer. The dissolved batch and 4.2 L of DI water are simultaneously charged to the heel slurry at constant rates over 6 hours to maintain a constant solvent composition in the crystallizer. Use of Intermig agitators during the crystallization is preferred. During this addition the slurry is wet-milled when the crystal lengths become excessively long or the slurry becomes too thick. The slurry is aged for 2 to 16 hours until the product concentration in the supernatant remains constant. The slurry is filtered to isolate a crystalline wet cake. The wet cake is washed with 1 to 2 bed volumes of 30 % 2-propanol in water and then twice with 1 bed volume of DI water each. The washed wet cake is dried under vacuum at 50°C.

### EXAMPLE 7

Preparation of Amino Alcohol 3 and ee Upgrading-- Through Process

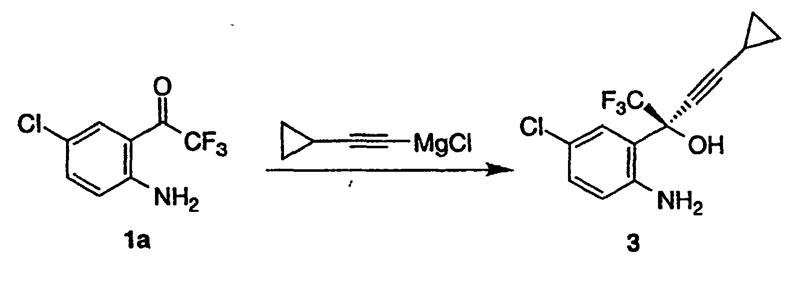

| Materials | Amount | Mol | MW |
|---|---|---|---|
| Ketone **1** | 1.00 kg | 4.47 | 223.58 |
| (1R, 2S)-N-Pyrrolidinyl norephedrine | 1.35 kg | 6.58 | 205.30 |
| Cyclopropyl acetylene | 361.9 g | 5.47 | 66.10 |
| n-BuMgCI (2.0 M in THF) | 2.68 L | 5.37 | |
| Trifluoroethanol (99%) | 429.5 g | 4.29 | 100.04 |
| ZnEt₂ (0.892 M in hexane) | 6.02 L | 5.37 | |
| THF | 9.36 L | | |
| 30% K₂CO₃ | 1.2 L | | |
| 1 M Citric acid | 3.5 L | | |
| Heptane | 12 L | | |
| Isopropyl acetate (IPAc) | 40 L | | |
| 12N HCl | 405 mL | 4.88 | |
| *tert*-Butyl methyl ether (MTBE) | 6 L | | |
| Toluene | 6.25 L | | |
| Na₂CO₃ | 1.2 kg | 11.25 | |

A solution of diethyl zinc in hexane was added to a solution of trifluoroethanol (429.5 g, 4.29'mol) and (1R, 2S)-N-pyrrolidinyl norephedrine (1.35 kg, 6.58 mol) in THF (9 L), under nitrogen, at 0 °C. The resulting mixture was stirred at room temperature for approx. 30 min. In another dry flask a solution of chloromagnesiumcyclopropylacetylide was prepared as follows. To a solution of *n-*butylmagnesium chloride in THF (2 M, 2.68 L, 5.37 mol) was added neat cyclopropylacetylene at 0 °C keeping the temperature ≤ 25 °C. The solution was stirred at 0 °C for 1 ∼ 2 h. The solution of chloromagnesiumcyclopropylacetylide was then warmed to room temperature and was transferred into the zinc reagent *via* cannula over 5 min followed by vessel rinse with 0.36 L of THF. The resulting mixture was aged at - 30 °C for 0.5 h and was then cooled to 20 °C. The ketoaniline **1** (1.00 kg, 4.47 mol) was added in one portion as a solid, and the resulting mixture was stirred at 20-28 °C for 3 h.

The reaction was quenched with 30% aq. potassium carbonate (1.2 L) and aged for 1 h. The solid waste was filtered and the cake was washed with THF (3 cake volumes). The filtrate and wash were combined and solvent switched to IPAc.

The IPAc solution of product **3** and pyrrolidinyl norephedrine was washed with citric acid (3.5 L) and with water (1.5 L). The combined aqueous layers were extracted with IPAc (2 L) and saved for norephedrine recovery. To the combined organic layers was added 12N HCl (405 mL, 4.88 mol), to form a thin slurry of the amino alcohol-HCl salt. The mixture was aged for 30 min at 25 °C and was then dried azeotropically.

The slurry was aged at 25 °C for 30 min and filtered. The cake was washed with 2.5 L of IPAc and dried at 25 °C under vacuum/nitrogen for 24 h to give 1.76 kg of the wet HCl salt.

The salt was dissolved in a mixture of MTBE (6 L) and aq Na₂CO₃ (1.18 kg in 6.25 L water). The layers were separated and the organic layer was washed with 1.25 L of water. The organic layer was then solvent switched into toluene.

Heptane (5 L) was added over 1 h at 25 °C. The slurry was cooled to 0 °C, aged for 1 h, and filtered. The solid was washed with heptane (2 cake volumes) and was dried to give 1.166 kg (90% overall yield) of amino alcohol **3** as a white crystalline solid.

### Norephedrine recovery

The aqueous solution was basified to pH13 using 50% aq NaOH, and extracted with heptane (2 L). The heptane solution was washed with water (1 L) and concentrated to remove residual IPAc and water. The final volume was adjusted to about 3 L. The heptane solution was cooled to -20 °C, aged for 2 h, and filtered. The solid was washed with cold heptane (1 cake volume) and dried to give 1.269 kg solid (94% recovery).

### EXAMPLE 8

50 % Drug-Loaded Compressed tablet Of Efavirenz

| **Ingredient** | **Amt per batch** |
|---|---|
| **Core Tablet:** | |
| efavirenz | 950 g |
| microcrystalline cellulose NF | 380 g |
| hydroxypropyl cellulose LF NF | 60.8 g |
| croscarmellose sodium | 95 g |
| sodium lauryl sulfate | 19 g |
| lactose hydrous spray dried (EG)* | 19.8 % w/w |
| magnesium stearate (EG)* | 1% w/w |
| water | 1.045 L |
| **Film Coating Material per Tablet:** | 3.3% by wt of tablet |
| hydroxypropyl cellulose LF NF | 8.54 mg (2.5 %) |
| hydroxypropyl methylcellulose USP 6CPS | 8.54 mg (2.5 %) |
| titanium dioxide USP | 3.42 mg (1 %) |
| water | (94 %) |

| | |
|---|---|
| * EG = extragranular | |

Efavirenz (950 g) was blended with microcrystalline cellulose (380 g), sodium lauryl sulfate (19 g), hydroxypropyl cellulose (60.8 g) and croscarmellose sodium (95 g) in a Fielder 10 L high shear granulator mixer for four minutes. At least about 1.1 weight % water per weight of efavirenz (1.045 L) was added to wet granulate the blended mixture over about 6 minutes to about 8 minutes to agglomerate the mixture using an appropriate spray nozzle. The granulated mixture is dried to a moisture content of about 2% to about 5% in a Glatt WST-15 fluid bed drier. The dried mixture was milled using a 40 G round screen in a Comil. The milled mixture was blended in a V-Blender with lactose for 4 minutes (calculated amount is the amount needed to make the final composition contain 19.8 % lactose by weight). The blended mixture was lubricated with magnesium stearate (calculated amount is the amount needed to make the final composition contain 1 % magnesium stearate by weight) in the V-Blender for 3 minutes. The lubricated mixture was compressed using a beta press to give a compressed tablet of the desired shape. The compressed tablets were film coated with an aqueous coating suspension that contains 2.5 % hydroxypropyl cellulose (HPC); 2.5 % hydroxymethylcellulose (HPMC); and 1 % titanium dioxide (TiO₂) and 94 % water by weight percent in a 19" O'Hara pan coater to a coat weight of about 3.3% per tablet. Note that the coat is the dried form of the suspension.

## Claims

1. A compressed tablet comprising: efavirenz, filler/disintegrant, superdisintegrant, binder, surfactant, a diluent/compression aid which comprises lactose, lubricant, and solvent, wherein the efavirenz is crystalline, which tablet is obtainable by a granulation process in which the superdisintegrant and disintegrant are added intragranularly and the lactose is added extragranularly and which tablet:
(i) comprises from 1 to 75% of efavirenz by weight of the total composition of the compressed tablet;
(a) providing the tablet comprises no more than 10% by weight of the total weight of superdisintegrant; or
(b) wherein the binder is a hydroxypropylcellulose; or
(ii) contains 600 mg of efavirenz in the total composition of the compressed tablet.

2. A compressed tablet, according to claim 1, which tablet is film coated with a suspension including combinations of one, two or three of the following: carboxymethylcellulose sodium, carnauba wax, cellulose acetate phthalate, cetyl alcohol, confectioner's sugar, ethyl cellulose, gelatine, hydroxyethylcellulose, hydroxypropylcellulose, hydroxypropylmethylcellulose, liquid glucose, maltodextrin, methylcellulose, microcrystalline wax, Opadry and Opadry II, polymethacrylates, polyvinyl alcohol, shellac, sucrose, talc, titanium dioxide, and zein.

3. A compressed tablet, as recited in Claim 2, where the film coating comprises: hydroxypropylcellulose, hydroxypropylmethylcellulose, and titanium dioxide.

4. A compressed tablet, as recited in Claim 2 or 3, wherein the concentration of the film coating is up to 10% of the drug amount.

5. A compressed tablet, as recited in Claim 4, wherein the concentration of the film coating is from 3.1% to 3.3% of the drug amount.

6. The compressed tablet, as recited in any preceding Claim,
wherein the binder comprises: acacia, alginic acid, carbomer, dextrin, ethylcellulose, gelatine, guar gum, hydroxyethylcellulose, hydroxypropylcellulose, hydroxypropylmethylcellulose, liquid glucose, magnesium aluminium silicate, maltodextrin, methylcellulose, polymethacrylates, povidone, pregelatinized starch,
sodium alginate, starch, or zein.

7. The compressed tablet, as recited in any preceding claim,
wherein the filler comprises: lactose, calcium carbonate, calcium sulphate, compressible sugars, dextrates, dextrin, dextrose, calcium phosphate, kaolin, magnesium carbonate, magnesium oxide, maltodextrin mannitol, powdered cellulose, pregelatinized starch, or sucrose.

8. The compressed tablet, as recited in any preceding claim,
wherein the disintegrant comprises: alginic acid, carboxymethylcellulose calcium, carboxymethylcellulose sodium, colloidal silicon dioxide, croscarmellose sodium, crospovidone, guar gum, magnesium aluminium silicate, methylcellulose, microcrystalline cellulose, polyacrilin potassium, powdered cellulose, pregelatinized starch, sodium alginate or starch.

9. The compressed tablet, as recited in any preceding claim,
wherein the superdisintegrant comprises: carboxymethylcellulose sodium, croscarmellose sodium, povidone, guar gum, polacrilin potassium or pregelatinized starch.

10. The compressed tablet, as recited in Claim 6, wherein the disintegrant is microcrystalline cellulose.

11. The compressed tablet, as recited in Claim 6 or 7, wherein the superdisintegrant is croscarmellose sodium.

12. The compressed tablet, as recited in any preceding Claim,
wherein the surfactant comprises: sodium lauryl sulphate, docusate sodium, benzalkonium chloride, benzethonium chloride, or cetrimide.

13. The compressed tablet, as recited in Claim 12, wherein the surfactant is sodium lauryl sulphate.

14. The compressed tablet, as recited in any preceding Claim,
wherein the diluent/compression aid further comprises: calcium carbonate, calcium sulphate, compressible sugars, confectioner's sugar, dextrates, dextrin, dextrose,
dibasic calcium phosphate dihydrate, glyceryl palmitostearate, hydrogenated vegetable oil (type I), kaolin, magnesium carbonate, magnesium oxide, maltodextrin, mannitol, polymethacrylates, potassium chloride, powdered cellulose, pregelatinized starch, sodium chloride, sorbitol, starch, sucrose, sugar, spheres, talc or tribasic calcium phosphate.

15. The compressed tablet, as recited in Claim 14, wherein the diluent/compression aid is lactose hydrous spray dried.

16. The compressed tablet, as recited in any preceding Claim,
wherein the lubricant comprises: calcium stearate, glyceryl monostearate, glyceryl palmitostearate, hydrogenated castor oil, hydrogenated vegetable oil, light mineral oil,
magnesium stearate, mineral oil, polyethylene glycol, sodium benzoate, sodium lauryl sulphate, sodium stearyl fumarate, stearic acid, talc or zinc stearate.

17. The compressed tablet, as recited in Claim 16, wherein the lubricant is magnesium stearate.

18. The compressed tablet, as recited in any preceding Claim,
wherein the solvent comprises: water, ethanol or mixtures thereof.

19. The compressed tablet, as recited in Claim 18, wherein the solvent is water.

20. The compressed tablet, as recited in Claim 1, comprising efavirenz, microcrystalline cellulose NF, hydroxypropylcellulose LF NF, croscarmellose sodium, sodium lauryl sulphate, lactose hydrous spray dried (EG), and magnesium stearate (EG).

21. The compressed tablet, as recited in any preceding Claim, which is made by a wet granulation process.

22. The compressed tablet, as recited in any one of Claims 1 to 20, which is made by a wet granulation process in which the efavirenz, filler/disintegrant, superdisintegrant, binder and surfactant are added intragranularly, and the diluent/compression aid and lubricant are added extragranularly.

23. The compressed tablet, as recited in any preceding Claim,
wherein efavirenz is about 50% by weight of the total composition of the compressed tablet.

24. The compressed tablet, as recited in any preceding Claim, but not Claim 1 (ii), which comprises 300 mg efavirenz.

25. The compressed tablet, as recited in Claim 24, containing about 300 mg of efavirenz, about 120 mg microcrystalline cellulose NF, about 19.2 mg hydroxypropyl cellulose LF NF, about 30 mg croscarmellose sodium, about 6 mg sodium lauryl sulfate, about 118.8 mg lactose hydrous spray dried (EG), and about 6 mg magnesium stearate (EG).

26. A process for the preparation of a 50% drug loaded compressed tablet comprising the following steps:
(a) blending crystalline efavirenz with a filler/disintegrant, super-dinsintegrant, binder and surfactant;
(b) adding at least 1.1% by weight of water per weight of efavirenz to wet granulate the blended mixture to agglomerate the mixture;
(c) drying the granulated mixture to a moisture content of about 0% to about 10%;
(d) milling the dried mixture to granulate to a uniform size;
(e) blending the milled mixture with a filler/compression aid which comprises lactose;
(f) lubricating the blended mixture with a lubricant; and
(g) compressing the lubricated mixture to a compressed tablet of the desired shape.

27. The process as recited in Claim 26 which comprises the additional step of film coating the compressed tablet with a film coating suspension to produce the desired film coated compressed tablet.

28. The process as recited in Claim 26 or 27 wherein the granulated mixture is dried to a moisture content of about 2% to about 5%.

29. A process according to Claims 26, 27 or 28 which further comprises
(h) film coating the compressed tablet with a film coating suspension to about 1% to about 10% by weight of the weight of compressed tablet.

30. The process as recited in Claim 29, wherein the blended mixture is wet granulated for about 6 minutes.

31. The process as recited in Claim 29 or 30, wherein the film coating suspension comprises hydroxypropylcellulose, hydroxypropylmethylcellulose and titanium dioxide.

32. The process as recited in Claim 29, 30 or 31, wherein the compressed tablet is film coated with the film coating suspension to about 3.1% to about 3.3% by weight of the weight of the compressed tablet.

## Patentansprüche

1. Eine gepresste Tablette, umfassend: Efavirenz, Füllstoff/Sprengmittel, Supersprengmittel, Bindemittel, oberflächenaktives Mittel, ein Verdünnungsmittel/Presshilfsmittel, das Lactose enthält, Gleitmittel und Lösungsmittel, wobei das Efavirenz kristallin ist, wobei die Tablette durch ein Granulierverfahren erhalten werden kann, bei dem das Supersprengmittel und das Sprengmittel intragranular und die Lactose extragranular zugegeben werden, und wobei die Tablette:
(i) 1 bis 75 Gew.-% Efavirenz, bezogen auf die Gesamtzusammensetzung der gepressten Tablette, enthält,
(a) sofern die Tablette nicht mehr als 10 Gew.-%, bezogen auf das Gesamtgewicht, an Supersprengmittel, enthält, oder
(b) wobei das Bindemittel eine Hydroxypropylcellulose ist, oder
(ii) 600 mg Efavirenz in der Gesamtzusammensetzung der gepressten Tablette enthält.

2. Eine gepresste Tablette gemäß Anspruch 1, wobei die Tablette filmbeschichtet ist mit einer Suspension, die Kombinationen aus einem, zwei oder drei der folgenden Stoffe enthält:
Carboxymethylcellulose-Natrium, Carnaubawachs, Celluloseacetatphthalat, Cetylalkohol, Puderzucker, Ethylcellulose, Gelatine, Hydroxyethylcellulose, Hydroxypropylcellulose, Hydroxypropylmethylcellulose, flüssige Glukose, Maltodextrin, Methylcellulose, mikrokristallines Wachs, Opadry und Opadry II, Polymethacrylate, Polyvinylalkohol, Schellack, Saccharose, Talk, Titandioxid und Zein.

3. Eine gepresste Tablette wie in Anspruch 2 angegeben, wobei die Filmbeschichtung umfasst: Hydroxypropylcellulose, Hydroxypropylmethylcellulose und Titandioxid.

4. Eine gepresste Tablette wie in Anspruch 2 oder 3 angegeben, wobei die Konzentration der Filmbeschichtung bis zu 10% der Arzneistoffmenge beträgt.

5. Eine gepresste Tablette wie in Anspruch 4 angegeben, wobei die Konzentration der Filmbeschichtung 3,1% bis 3,3% der Arzneistoffmenge beträgt.

6. Die gepresste Tablette wie in einem vorhergehenden Anspruch angegeben, wobei das Bindemittel umfasst: Akaziengummi, Alginsäure, Carbomer, Dextrin, Ethylcellulose, Gelatine, Guargummi, Hydroxyethylcellulose, Hydroxypropylcellulose, Hydroxypropylmethylcellulose, flüssige Glukose, Magnesiumaluminiumsilikat, Maltodextrin, Methylcellulose, Polymethacrylate, Povidon, vorgelatinierte Stärke, Natriumalginat, Stärke oder Zein.

7. Die gepresste Tablette wie in einem vorhergehenden Anspruch angegeben, wobei der Füllstoff umfasst: Lactose, Calciumcarbonat, Calciumsulfat, verpressbare Zucker, Dextrate, Dextrin, Dextrose, Calciumphosphat, Kaolin, Magnesiumcarbonat, Magnesiumoxid, Maltodextrin, Mannit, pulverförmige Cellulose, vorgelatinierte Stärke oder Saccharose.

8. Die gepresste Tablette wie in einem vorhergehenden Anspruch angegeben, wobei das Sprengmittel umfasst: Alginsäure, Carboxymethylcellulose-Calcium, Carboxymethylcellulose-Natrium, kolloidales Siliziumdioxid, Croscarmellose-Natrium, Crospovidon, Guargummi, Magnesiumaluminiumsilikat, Methylcellulose, mikrokristalline Cellulose, Polyacrilin-Kalium, pulverförmige Cellulose, vorgelatinierte Stärke, Natriumalginat oder Stärke.

9. Die gepresste Tablette wie in einem vorhergehenden Anspruch angegeben, wobei das Supersprengmittel umfasst: Carboxymethylcellulose-Natrium, Croscarmellose-Natrium, Povidon, Guargummi, Polacrilin-Kalium oder vorgelatinierte Stärke.

10. Die gepresste Tablette wie in Anspruch 6 angegeben, wobei das Sprengmittel mikrokristalline Cellulose ist.

11. Die gepresste Tablette wie in Anspruch 6 oder 7 angegeben, wobei das Supersprengmittel Croscarmellose-Natrium ist.

12. Die gepresste Tablette wie in einem vorhergehenden Anspruch angegeben, wobei das oberflächenaktive Mittel umfasst: Natriumlaurylsulfat, Docusat-Natrium, Benzalkoniumchlorid, Benzethoniumchlorid oder Cetrimid.

13. Die gepresste Tablette wie in Anspruch 12 angegeben, wobei das oberflächenaktive Mittel Natriumlaurylsulfat ist.

14. Die gepresste Tablette wie in einem vorhergehenden Anspruch angegeben, wobei das Verdünnungsmittel/Presshilfsmittel ferner umfasst: Calciumcarbonat, Calciumsulfat, verpressbare Zucker, Puderzucker, Dextrate, Dextrin, Dextrose, zweibasisches Calciumphosphat-Dihydrat, Glycerylpalmitostearat, hydriertes Pflanzenöl (Typ I), Kaolin, Magnesiumcarbonat, Magnesiumoxid, Maltodextrin, Mannit, Polymethacrylate, Kaliumchlorid, pulverförmige Cellulose, vorgelatinierte Stärke, Natriumchlorid, Sorbit, Stärke, Saccharose, Zucker, Kugeln, Talk oder dreibasisches Calciumphosphat.

15. Die gepresste Tablette wie in Anspruch 14 angegeben, wobei das Verdünnungsmittel/Presshilfsmittel sprühgetrocknete wasserhaltige Lactose ist.

16. Die gepresste Tablette wie in einem vorhergehenden Anspruch angegeben, wobei das Gleitmittel umfasst: Calciumstearat, Glycerylmonostearat, Glycerylpalmitostearat, hydriertes Rizinusöl, hydriertes Pflanzenöl, leichtes Mineralöl, Magnesiumstearat, Mineralöl, Polyethylenglycol, Natriumbenzoat, Natriumlaurylsulfat, Natriumstearylfumarat, Stearinsäure, Talk oder Zinkstearat.

17. Die gepresste Tablette wie in Anspruch 16 angegeben, wobei das Gleitmittel Magnesiumstearat ist.

18. Die gepresste Tablette wie in einem vorhergehenden Anspruch angegeben, wobei das Lösungsmittel umfasst: Wasser, Ethanol oder Mischungen davon.

19. Die gepresste Tablette wie in Anspruch 18 angegeben, wobei das Lösungsmittel Wasser ist.

20. Die gepresste Tablette wie in Anspruch 1 angegeben, umfassend Efavirenz, mikrokristalline Cellulose NF, Hydroxypropylcellulose LF NF, Croscarmellose-Natrium, Natriumlaurylsulfat, sprühgetrocknete wasserhaltige Lactose (EG) und Magnesiumstearat (EG).

21. Die gepresste Tablette wie in einem vorhergehenden Anspruch angegeben, die durch ein Nassgranulierverfahren erzeugt wird.

22. Die gepresste Tablette wie in einem der vorhergehenden Ansprüche 1 bis 20 angegeben, die durch ein Nassgranulierverfahren erzeugt wird, bei dem das Efavirenz, der Füllstoff/Sprengmittel, das Supersprengmittel, das Bindemittel und das oberflächenaktive Mittel intragranular zugegeben werden und das Verdünnungsmittel/Presshilfsmittel und das Gleitmittel extragranular zugegeben werden.

23. Die gepresste Tablette wie in einem vorhergehenden Anspruch angegeben, wobei das Efavirenz etwa 50 Gew.-% der Gesamtzusammensetzung der gepressten Tablette ausmacht.

24. Die gepresste Tablette wie in einem vorhergehenden Anspruch, nicht jedoch Anspruch 1 (ii), angegeben, die 300 mg Efavirenz enthält.

25. Die gepresste Tablette wie in Anspruch 24 angegeben, die etwa 300 mg Efavirenz, etwa 120 mg mikrokristalline Cellulose NF, etwa 19,2 mg Hydroxypropylcellulose LF NF, etwa 30 mg Croscarmellose-Natrium, etwa 6 mg Natriumlaurylsulfat, etwa 118,8 mg wasserhaltige sprühgetrocknete Lactose (EG) und etwa 6 mg Magnesiumstearat (EG) enthält.

26. Ein Verfahren zur Herstellung einer mit 50% Arzneimittel beladenen gepressten Tablette, das die folgenden Schritte umfasst:
(a) Vermischen von kristallinem Efavirenz mit Füllstoff/Sprengmittel, Supersprengmittel, Bindmittel und oberflächenaktivem Mittel,
(b) Zugeben von wenigstens 1,1 Gew.-% Wasser pro Gewicht Efavirenz, um die vermischte Mischung nasszugranulieren, um die Mischung zu agglomerieren,
(c) Trocknen der granulierten Mischung bis zu einem Feuchtigkeitsgehalt von etwa 0% bis etwa 10%,
(d) Vermahlen der getrockneten Mischung, um sie zu einer einheitlichen Größe zu granulieren,
(e) Vermischen der gemahlenen Mischung mit einem Füllstoff/Presshilfsmittel, das Lactose enthält,
(f) Schmieren der vermischten Mischung mit einem Gleitmittel und
(g) Pressen der geschmierten Mischung zu einer gepressten Tablette mit der erwünschten Form.

27. Das Verfahren wie in Anspruch 26 angegeben, das den zusätzlichen Schritt der Filmbeschichtung der gepressten Tablette mit einer Filmbeschichtungssuspension umfasst, um die erwünschte filmbeschichtete gepresste Tablette zu erzeugen.

28. Das Verfahren wie in Anspruch 26 oder 27 angegeben, wobei die granulierte Mischung bis zu einem Feuchtigkeitsgehalt von etwa 2% bis etwa 5% getrocknet wird.

29. Ein Verfahren gemäß Anspruch 26, 27 oder 28, das ferner umfasst (h) Filmbeschichten der gepressten Tablette mit einer Filmbeschichtungssuspension bis etwa 1 Gew.-% bis etwa 10 Gew.-% des Gewichts der gepressten Tablette.

30. Das Verfahren wie in Anspruch 29 angegeben, wobei die vermischten Mischung etwa 6 Minuten lang nassgranuliert wird.

31. Das Verfahren wie in Anspruch 29 oder 30 angegeben, wobei die Filmbeschichtungssuspension Hydroxypropylcellulose, Hydroxypropylmethylcellulose und Titandioxid enthält.

32. Das Verfahren wie in Anspruch 29, 30 oder 31 angegeben, wobei die gepresste Tablette mit der Filmbeschichtungssuspension bis etwa 3,1 Gew.-% bis etwa 3,3 Gew.-% des Gewichts der gepressten Tablette, filmbeschichtet wird.

## Revendications

1. Comprimé comprenant: de l'efavirenz, une charge/un délitant, un superdélitant, un liant, un tensioactif, un diluant/un auxiliaire de compression qui comprend du lactose, un lubrifiant et un solvant, où l'efavirenz est cristallin, lequel comprimé peut être obtenu par un procédé de granulation dans lequel le superdélitant et le délitant sont ajoutés en phase intra-granulaire et le lactose est ajouté en phase extra-granulaire et lequel comprimé:
(i) comprend de 1 à 75% d'efavirenz en poids de la composition totale du comprimé;
(a) à condition que le comprimé ne comprenne pas plus de 10% en poids du poids total du superdélitant; ou
(b) où le liant est une hydroxypropylcellulose; ou bien
(ii) contient 600 mg d'efavirenz dans la composition totale du comprimé.

2. Comprimé selon la revendication 1, lequel comprimé est pelliculé avec une suspension incluant des combinaisons d'un, deux ou trois des éléments suivants:
carboxyméthylcellulose sodique, cire de carnauba, phtalate d'acétate de cellulose, alcool cétylique, sucre de confiseur, éthylcellulose, gélatine, hydroxyéthylcellulose, hydroxypropylcellulose, hydroxypropylméthylcellulose, glucose liquide, maltodextrine, méthylcellulose, cire microcristalline, Opadry et Opadry II, polyméthacrylates, alcool polyvinylique, gomme-laque, saccharose, talc, dioxyde de titane et zéine.

3. Comprimé selon la revendication 2, dans lequel le pelliculage comprend: de l'hydroxypropylcellulose, de l'hydroxypropylméthylcellulose et du dioxyde de titane.

4. Comprimé selon la revendication 2 ou 3, dans lequel la concentration du pelliculage représente jusqu'à 10% de la quantité de médicament.

5. Comprimé selon la revendication 4, dans lequel la concentration du pelliculage représente de 3,1% à 3,3% de la quantité de médicament.

6. Comprimé selon l'une quelconque des revendications précédentes, dans lequel le liant comprend: de la gomme arabique, de l'acide alginique, du carbomère, de la dextrine, de l'éthylcellulose, de la gélatine, de la gomme de guar, de l'hydroxyéthylcellulose, de l'hydroxypropylcellulose, de l'hydroxypropylméthylcellulose, du glucose liquide, du silicate de magnésium et d'aluminium, de la maltodextrine, de la méthylcellulose, des polyméthacrylates, de la povidone, de l'amidon pré-gélatinisé, de l'alginate de sodium, de l'amidon ou de la zéine.

7. Comprimé selon l'une quelconque des revendications précédentes, dans lequel la charge comprend: du lactose, du carbonate de calcium, du sulfate de calcium, des sucres compressibles, des dextrates, de la dextrine, du dextrose, du phosphate de calcium, du kaolin, du carbonate de magnésium, de l'oxyde de magnésium, de la maltodextrine, du mannitol, de la cellulose pulvérisée, de l'amidon pré-gélatinisé ou du saccharose.

8. Comprimé selon l'une quelconque des revendications précédentes, dans lequel le délitant comprend: de l'acide alginique, de la carboxyméthylcellulose calcique, de la carboxyméthylcellulose sodique, de la silice colloïdale, de la croscarmellose sodique, de la crospovidone, de la gomme de guar, du silicate de magnésium et d'aluminium, de la méthylcellulose, de la cellulose microcristalline, de la polyacriline potassique, de la cellulose pulvérisée, de l'amidon pré-gélatinisé, de l'alginate de sodium ou de l'amidon.

9. Comprimé selon l'une quelconque des revendications précédentes, dans lequel le superdélitant comprend: de la carboxyméthylcellulose sodique, de la croscarmellose sodique, de la povidone, de la gomme de guar, de la polyacriline potassique ou de l'amidon pré-gélatinisé.

10. Comprimé selon la revendication 6, dans lequel le délitant est de la cellulose microcristalline.

11. Comprimé selon la revendication 6 ou 7, dans lequel le superdélitant est de la croscarmellose sodique.

12. Comprimé selon l'une quelconque des revendications précédentes, dans lequel le tensioactif comprend: du laurylsulfate de sodium, du docusate sodique, du chlorure de benzalkonium, du chlorure de benzéthonium ou du cétrimide.

13. Comprimé selon la revendication 12, dans lequel le tensioactif est du laurylsulfate de sodium.

14. Comprimé selon l'une quelconque des revendications précédentes, dans lequel le diluant/auxiliaire de compression comprend en outre: du carbonate de calcium, du sulfate de calcium, des sucres compressibles, du sucre de confiseur, des dextrates, de la dextrine, du dextrose, du phosphate de calcium dibasique dihydraté, du palmitostéarate de glycéryle, de l'huile végétale hydrogénée (type I), du kaolin, du carbonate de magnésium, de l'oxyde de magnésium, de la maltodextrine, du mannitol, des polyméthacrylates, du chlorure de potassium, de la cellulose pulvérisée, de l'amidon pré-gélatinisé, du chlorure de sodium, du sorbitol, de l'amidon, du saccharose, du sucre, des sphères, du talc ou du phosphate de calcium tribasique.

15. Comprimé selon la revendication 14, dans lequel le diluant/auxiliaire de compression est une pulvérisation de lactose hydraté séché.

16. Comprimé selon l'une quelconque des revendications précédentes, dans lequel le lubrifiant comprend: du stéarate de calcium, du monostéarate de glycéryle, du palmitostéarate de glycéryle, de l'huile de ricin hydrogénée, de l'huile végétale hydrogénée, de l'huile minérale légère, du stéarate de magnésium, de l'huile minérale, du polyéthylène glycol, du benzoate de sodium, du laurylsulfate de sodium, du stéarylfumarate de sodium, de l'acide stéarique, du talc ou du stéarate de zinc.

17. Comprimé selon la revendication 16, dans lequel le lubrifiant est du stéarate de magnésium.

18. Comprimé selon l'une quelconque des revendications précédentes, dans lequel le solvant comprend: de l'eau, de l'éthanol ou des mélanges de ceux-ci.

19. Comprimé selon la revendication 18, dans lequel le solvant est de l'eau.

20. Comprimé selon la revendication 1, comprenant de l'efavirenz, de la cellulose microcristalline NF, de l'hydroxypropylcellulose LF NF, de la croscarmellose sodique, du laurylsulfate de sodium, une pulvérisation de lactose hydraté séché (EG) et du stéarate de magnésium (EG).

21. Comprimé selon l'une quelconque des revendications précédentes, qui est fait par un procédé de granulation par voie humide.

22. Comprimé selon l'une quelconque des revendications 1 à 20, qui est fait par un procédé de granulation par voie humide dans lequel l'efavirenz, la charge/le délitant, le superdélitant, le liant et le tensioactif sont ajoutés en phase intra-granulaire et le diluant/auxiliaire de compression et le lubrifiant sont ajoutés en phase extra-granulaire.

23. Comprimé selon l'une quelconque des revendications précédentes, dans lequel l'efavirenz représente environ 50% en poids de la composition totale du comprimé.

24. Comprimé selon l'une quelconque des revendications précédentes à l'exception de la revendication 1 (ii), lequel comprend 300 mg d'efavirenz.

25. Comprimé selon la revendication 24, contenant environ 300 mg d'efavirenz, environ 120 mg de cellulose microcristalline NF, environ 19,2 mg d'hydroxypropylcellulose LF NF, environ 30 mg de croscarmellose sodique, environ 6 mg de laurylsulfate de sodium, environ 118,8 mg de pulvérisation de lactose hydraté séché (EG) et environ 6 mg de stéarate de magnésium (EG).

26. Procédé de préparation d'un comprimé chargé à 50% de médicament, comprenant les étapes consistant à:
(a) mélanger de l'efavirenz cristallin avec une charge/un délitant, un superdélitant, un liant et un tensioactif;
(b) ajouter au moins 1,1% en poids d'eau par poids d'efavirenz pour granuler par voie humide le mélange afin d'agglomérer le mélange;
(c) sécher le mélange granulé à une teneur en humidité d'environ 0% à environ 10%;
(d) moudre le mélange séché pour le granuler en une taille uniforme;
(e) mélanger le mélange moulu avec une charge/auxiliaire de compression qui comprend du lactose;
(f) lubrifier le mélange avec un lubrifiant; et
(g) comprimer le mélange lubrifié en un comprimé de la forme désirée.

27. Procédé selon la revendication 26, qui comprend l'étape supplémentaire consistant à pelliculer le comprimé avec une suspension de pelliculage pour produire le comprimé pelliculé désiré.

28. Procédé selon la revendication 26 ou 27, dans lequel le mélange granulé est séché à une teneur en humidité d'environ 2% à environ 5%.

29. Procédé selon la revendication 26, 27 ou 28, qui comprend en outre:
pelliculer le comprimé avec une suspension de pelliculage à raison d'environ 1% à environ 10% en poids du poids du comprimé.

30. Procédé selon la revendication 29, dans lequel le mélange est granulé par voie humide pendant environ 6 minutes.

31. Procédé selon la revendication 29 ou 30, dans lequel la suspension de pelliculage comprend de l'hydroxypropylcellulose, de l'hydroxypropylméthylcellulose et du dioxyde de titane.

32. Procédé selon la revendication 29, 30 ou 31, dans lequel le comprimé est pelliculé avec la suspension de pelliculage à raison d'environ 3,1% à environ 3,3% en poids du poids du comprimé.
